(19) Europäisches Patentamt
European Patent Office
Office européen des brevets

(11) Publication number: 0 136 704
B1

(12) EUROPEAN PATENT SPECIFICATION

(45) Date of publication of patent specification: 13.12.89

(51) Int. Cl.$^4$: A 61 K 7/043

(21) Application number: 84111810.2

(22) Date of filing: 03.10.84

# (54) Nail enamel containing silicone-coated pigments.

(30) Priority: 03.10.83 US 538601

(43) Date of publication of application:
10.04.85 Bulletin 85/15

(45) Publication of the grant of the patent:
13.12.89 Bulletin 89/50

(84) Designated Contracting States:
AT BE CH DE FR GB IT LI LU NL SE

(56) References cited:
EP-A-0 133 963
FR-A-2 205 557

CHEMICAL ABSTRACTS, vol. 94, no. 6, February 1981, page 358, no. 36125z, Columbus, Ohio, US; & JP - A - 80 136 213 (POLAR CHEMICALS LTD.) 23-10-1980

SEIFEN-OLE-FETTE-WACHSE, vol. 109, no. 9, June 1983, page 271, Augsburg, DE; "Filmbildner und Weichmacher für Nagellacke"

(73) Proprietor: Revlon, Inc.
767 Fifth Avenue
New York, N.Y.10022 (US)

(72) Inventor: Socci, Robert L.
21 Little Falls Road
Cedar Grove New Jersey (US)
Inventor: Ismailer, Anatoly A.
105-34 65th Avenue
Forest Hills, N.Y. (US)
Inventor: Castrogiovanni, Anthony
171 13th Street
Belford New Jersey (US)

(74) Representative: Körber, Wolfhart, Dr. et al
Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer
Steinsdorfstrasse 10
D-8000 München 22 (DE)

(56) References cited:
PATENTS ABSTRACTS OF JAPAN, vol. 8, no. 273 (C-256)1710r, 13th December 1984; & JP - A - 59 144 709 (POLA KASEI KOGYO K.K.) 18-08-1984

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 136 704 B1

Courier Press, Leamington Spa, England.

## Description

This invention relates to nail enamels. It particularly relates to nail enamels which are substantially free of the settling and the migration of the pigment and other materials suspended in the composition.

Conventional nail enamel compositions incorporate in their formulas a montmorillonite clay as a gellant to suspend pigments and pearlescent materials contained therein.

To achieve desired properties for these systems, as much as 2.0% or more of the gellant is recommended to effect complete and stable suspension. The use of larger amounts of the gellant in a nail enamel composition adversely affects the application and flow properties of the preparation. When the levels of the gellant are reduced to about 0.5—1.25%, other additives must then be added to maintain desired suspension and performance characteristics. It is also known that nail enamel compositions exhibit pigment migration, i.e., preferential separation, flocculation and flotation as distinguished from settling. While migration does affect the performance of the nail enamel, the streaking effects noticeable from such migration are aesthetically undesirable.

The publication "Chemical abstracts", Vol. 94 (1981) Feb. No. 6, No. 36125z discloses a water repellent-coated powder for cosmetics based on inorganic pigments such as talc, celite, $TiO_2$, Mg silicate, red Fe oxide and synthetic micra coated with methylhydrodienepolysiloxanes.

The EP—A—133 963 discloses a cosmetic composition containing inorganic pigment coated with an organosiloxane. Unexpectedly high amounts of pigments (40—60 wt.%) are incorporated in pigmented cosmetic products such as eyeshadows, foundations, moisturizers, and skin protectants.

Said publications do not discuss how to use specific inorganic pigments in the preparation of a superior nail enamel. U.S. Patent No. 4,166,110 discloses a nail enamel which contains an organically substituted polysiloxane to render the enamel film easily strippable from the nail. The patent does not discuss how to incorporate pigments into such a formulation and does not suggest coating pigments with the polysiloxane.

An article by B. V. Ashmead et al., "The Silicone Treatment of Titania", J. Oil, Col. Chem. Assoc., Vol. 54 (1971), pp. 403—424, discusses the adsorption of various silicone polymers onto the surface of titanium dioxide and discloses that silicone treated pigments disperse better in conventional paint media than untreated pigments. This disclosure does not suggest how coated pigments would behave in the solvent system employed in nail enamels. Moreover, the emphasis on dispersion, i.e. the length of time one needs to stir the paint in order to disperse the pigment in the surrounding media, does not support any suggestion as to how quickly the pigment settles and/or migrates once stirring is stopped. Indeed, freedom from settling and migration is of little or no concern to paint manufacturers, because the paint is in a non-transparent can until the user opens the can, stirs the paint, and applies the paint. By contrast, freedom from settling and migration even without stirring are of vital concern to the nail enamel manufacturer, who customarily sells the enamel in a transparent bottle, because the customer must be persuaded to buy the enamel based on how the enamel looks in the bottle.

In accordance with the present invention, a pigmented nail enamel is provided which is substantially free from settling and migration of the pigment contained therein, in which the nail enamel contains an inorganic pigment which is coated with a poly(organosiloxane). The improved nail enamel is prepared by coating the pigment with the polysiloxane before mixing the coated pigment with the remaining components of the nail enamel.

As indicated, the pigmented nail enamels contain pigment which has been coated with organically substituted polysiloxane.

The polysiloxane coating is generally characterized in that it imparts to the pigment the desirable properties mentioned herein when formulated into a nail enamel. While we do not intend to be bound by any particular specific definition for the coating, we use the term "polysiloxane" to include polymeric chains having up to 100 or even to 1,000 repeating (Si—O) units, wherein at least one Si atom of each chain is linked to the pigment surface or the coated pigment surface as hereinafter described through an oxygen atom. The chains can be cross-linked to each other as well. The remaining functional sites of each Si atom in the chain can be occupied by hydrogen, methyl, $C_2$—$C_{30}$ alkyl or alkenyl, and/or phenyl, and equivalents thereof resulting in units such as

$$-\!\!\left(-Si(CH_3)(C_6H_5)O-\right)\!\!-,\quad -\!\!\left(-Si(CH_3)(H)O-\right)\!\!-,\quad \text{or}\quad -\!\!\left(-Si(H)(C_6H_5)O-\right)\!\!-,$$

and generally capped with

$$—Si(CH_3)_3.$$

Satisfactory colors can be obtained in a variety of poly(organosiloxane)-coated pigments from Clarks Colors for whom the sole known distributor is Whittaker, Clark & Daniels, Inc. The product has the trade name "Hydrophobes". Alternatively, one can make the coated pigment by, for instance, reacting the uncoated pigment with (A) a silane of the formula $A_1SiX_1X_2X_3$, wherein $A_1$ is alkyl or alkenyl having up to 30 carbon atoms and $X_1$, $X_2$ and $X_3$ are independently chloro, methoxy or ethoxy

(B) $(CH_3)_3SiO -\!\!\!+ Si(CH_3)_2O -\!\!\!+_n Si(CH_3)_2OA_2$

wherein n is 1 to 100 or up to 1,000, and $A_2$ is $C_1$—$C_{30}$ alkyl; or

(C) $(CH_3)_3SiO -\!\!\!+ Si(CH_3)(H)O -\!\!\!+_p Si(CH_3)_3$

wherein p is 1 to 100 or up to 1,000. The reaction conditions are chosen so that $X_1$, $X_2$, $X_3$, $A_2$, or (H), as the case may be, hydrolyzes and bonds to the coated pigment surface through oxygen atoms. This mechanism, and appropriate reaction conditions, are familiar matters to the chemist.

The pigment which is incorporated within the coating can be any cosmetically acceptable inorganic or organic pigment. Examples of such pigments include: iron oxides, titanated mica, iron oxide coated mica, titanium dioxide (e.g. rutile, anatase), ultramarine, chromium oxide, chromium hydroxide, manganese violet, and their equivalents. Preferred pigments from the above list are iron oxide and titanium dioxide.

The poly(organosiloxane) coated pigment can in general comprise up to 10 wt.% of the nail enamel product. More preferably, the coated pigment comprises up to about 2 wt.%, and even more preferably it comprises about 1 wt.% to 2 wt.%.

Nail enamels which contain the coated pigments set forth above exhibit greatly reduced settling and migration compared to enamels which are identical except for lacking the silicone coating on the pigment.

In addition to the coated pigment, the nail enamel contains an otherwise conventional base, including nitrocellulose, solvent, and suspending agent. The base can also include a resin and a plasticizer.

The resin should be one which adds to the durability, adhesion, and gloss of the enamel. Examples of preferred resins include toluene sulfonamide formaldehyde resin, and polyesters. The plasticizer should be effective to aid the enamel coating's flexibility. Examples of preferred plasticizers include dibutyl phthalate and dioctyl phthalate. The solvent should dissolve the other components of the base, allowing the enamel to flow onto the nail, and should be able to evaporate from the nail. Examples of preferred solvents include toluene, isopropanol, butyl acetate, ethyl acetate, and mixtures thereof. The suspending agent should help suspend the pigments in the base. Examples of preferred suspending agents include montmorillonite clays.

A more complete listing of conventional bases which are useful with this invention can be found in standard sources well-known to the chemist, such as *Cosmetics, Science and Technology*, Balsam and Sagarin (eds.), 2nd Edition (1972), Vol. 2, Chapter 29 (Wiley Interscience, New York, N.Y.).

The coated pigment can be dispersed into the other nail enamel components readily by conventional mixing techniques employed in nail enamel manufacture, for example, chipping, roller milling, etc.

The superior effect of the present invention is seen in the following comparative examples. These examples are illustrative only and should not be construed as limiting. The numerical evaluation ratings have the following meanings:

5. Excellent—no visible signs of pigment migration or settling.
4. Good—Very slight settling observed, no signs of pigment migration.
3 Average—Some settling and very slight pigment migration.
2. Poor—Settling and migration readily apparent (not saleable).
1. Unacceptable—Very bad settling and migration (not saleable).

In the following tables where pigment is indicated as "treated" it had been coated with poly(methylhydrogen)siloxane obtained from Clark Colors with the product number 9454 for the coated iron oxide, and product number 9428 for the coated titanium dioxide. The amounts of treated pigment were chosen to provide equal amounts of pigment *per se* in all four samples.

All amounts are in percent by weight.

Example 1

Four samples of a nail enamel ("Ripe Plum") were prepared by mixing uniformly the ingredients indicated in Table 1. The products were compared for settling and migration after holding for two months without agitation, at room temperature or at 48.89°C.

TABLE 1

| Sample No. | 1 | 2 | 3 | 4 |
|---|---|---|---|---|
| Nitrocellulose | 15.00 | 15.00 | 15.00 | 15.00 |
| Dibutylphthalate | 4.50 | 4.50 | 4.50 | 4.50 |
| Toluene sulfonamide formaldehyde resin | 6.00 | 6.00 | 6.00 | 6.00 |
| Butyl acetate | 30.00 | 30.00 | 30.00 | 30.00 |
| Ethyl acetate | 8.00 | 8.00 | 8.00 | 8.00 |
| Toluene | 26.00 | 26.00 | 26.00 | 26.00 |
| Isopropanol | 7.35 | 7.35 | 7.59 | 7.59 |
| Bentone 27 (montmorillorite) | 1.00 | 1.00 | 1.00 | 1.00 |
| Titanium dioxide (treated) | 1.00 | 1.00 | — | — |
| Iron oxide (treated) | 0.48 | — | 0.48 | — |
| Titanium dioxide (untreated) | — | — | 0.76 | 0.76 |
| Iron oxide (untreated) | — | 0.48 | — | 0.48 |
| Untreated organic pigments (mixture of D&C Red 7 and ferric ammonium ferrocyanide) | 0.67 | 0.67 | 0.67 | 0.67 |
| Observation after 2 mos. | | | | |
| —at room temp. | 5 | 4 | 4 | 4 |
| —at 48.89°C | 5 | 3 | 3 | 3 |

Example 2

Four samples of a nail enamel ("Carmel") were prepared by mixing uniformly the ingredients indicated in Table 2. The products were compared for settling and migration after holding for two months without agitation, at room temperature or at 48.89°C.

TABLE 2

| Sample No. | 1 | 2 | 3 | 4 |
|---|---|---|---|---|
| Nitrocellulose | 15.00 | 15.00 | 15.00 | 15.00 |
| Dibutylphthalate | 4.50 | 4.50 | 4.50 | 4.50 |
| Toluene sulfonamide formaldehyde resin | 6.00 | 6.00 | 6.00 | 6.00 |
| Butyl acetate | 30.00 | 30.00 | 30.00 | 30.00 |
| Ethyl acetate | 8.00 | 8.00 | 8.00 | 8.00 |
| Toluene | 26.00 | 26.00 | 26.00 | 26.00 |
| Isopropanol | 8.327 | 8.327 | 9.517 | 9.517 |
| Bentone 27 (montmorillorite) | 1.00 | 1.00 | 1.00 | 1.00 |
| Titanium dioxide (treated) | 0.79 | 0.79 | — | — |
| Iron oxide (treated) | 0.35 | — | 0.35 | — |
| Titanium dioxide (untreated) | — | — | 0.60 | 0.60 |
| Iron oxide (untreated) | — | 0.35 | — | 0.35 |
| Untreated organic pigments (mixture of D&C Yellow 5, D&C Red 6, and ferric ammonium ferrocyanide) | 0.033 | 0.033 | 0.033 | 0.033 |
| Observation after 2 mos. | | | | |
| —at room temp. | 5 | 4 | 4 | 4 |
| —at 48.89°C | 5 | 3 | 3 | 3 |

The observations in Tables 1 and 2 indicate that nail enamels in which all inorganic pigments were coated with the organopolysiloxane were superior to enamels in which some, or all, inorganic pigments were not so coated.

**Claims**

1. A nail enamel comprising

a) an inorganic pigment selected from iron oxides, titanated mica, iron oxide coated mica, titanium

dioxide, ultramarine, chromium oxide, chromium hydroxide, and manganese violet, wherein said inorganic pigment is precoated with a polyorganosiloxane, including polymeric chains having up to 100 or even 1,000 repeating (Si—O) units, wherein the chains are cross-linked to each other, the remaining function sites of each Si-atom in the chain are occupied by hydrogen methyl, $C_2$—$C_{30}$ alkyl or alkenyl, and/or phenyl, resulting in units such as

$$-\!\!\left(Si(CH_3)(C_6H_5)O\right)\!\!-, \quad -\!\!\left(Si(CH_3)(H)O\right)\!\!-, \text{ or } -\!\!\left(Si(H)(C_6H_5)O\right)\!\!-,$$

and generally capped with

$$-Si(CH_3)_3,$$

wherein said precoating is provided by reacting the uncoated pigment with a silicone material selected from

(A) a silane of the formula $A_1SiX_1X_2X_3$, wherein $A_1$ is alkyl or alkenyl having up to 30 carbon atoms and $X_1$, $X_2$ and $X_3$ are independently chloro, methoxy or ethoxy,

(B) $(CH_3)_3SiO\!-\!\!\left(Si(CH_3)_2O\right)_n\!\!-\,Si(CH_3)_2OA_2$ wherein n is 1 to 100 or up to 1,000, and $A_2$ is $C_1$—$C_{30}$ alkyl, or

(C) $(CH_3)_3SiO\!-\!\!\left(Si(CH_3)(H)O\right)_p\!\!-\,Si(CH_3)_3$, wherein p is 1 to 100 or up to 1,000,

so that at least one Si-atom of each chain is linked to the pigment surface or the coated pigment surface through an oxygen atom, wherein the precoated pigment comprises up to 10 wt.% of the nail enamel and

b) a base comprising nitrocellulose, solvent and a suspending agent and optionally also including a resin and a plasticizer.

2. The nail enamel of Claim 1, wherein the polyorganosiloxane is polymethylhydrogensiloxane.

3. The nail enamel of Claims 1 and 2, wherein the precoated pigment comprises up to about 2 wt.% of the nail enamel.

4. The nail enamel of Claims 1 to 3, wherein the precoated pigment comprises about 1 wt.% to about 2 wt.%.

5. The nail enamel of Claims 1 to 4, wherein the inorganic pigment is selected from iron oxide and titanium dioxide.

6. A process for preparing a nail enamel comprising the steps of:

a) precoating an inorganic pigment selected from iron oxides, titanium mica, iron oxide coated, mica, titanium dioxide, ultramarine, chromium oxide, chromium hydroxide and manganese violet, with a polyorganosiloxane including polymeric chains having up to 100 or even to 1,000 repeating (Si—O) units, wherein the chains are cross-linked to each other, the remaining functional sites of each Si-atom in the chain are occupied by hydrogen, methyl, $C_2$—$C_{30}$ alkyl or alkenyl, and/or phenyl, resulting in units such as

$$-\!\!\left(Si(CH_3)\ (C_6H_5)O\right)\!\!-, \quad -\!\!\left(Si(CH_3)\ (H)O\right)\!\!-, \text{ or } -\!\!\left(Si(H)\ (C_6H_5)O\right)\!\!-,$$

and generally capped with

$$-Si(CH_3)_3,$$

wherein said precoating is provided by reacting the uncoated pigment with a silicone material selected from

(A) a silane of the formula $A_1SiX_1X_2X_3$, wherein $A_1$ is alkyl or alkenyl having up to 30 carbon atoms and $X_1$, $X_2$ and $X_3$ are independently chloro, methoxy or ethoxy,

(B) $(CH_3)_3SiO\,-\!\!\left(Si(CH_3)_2O\right)_n\!\!-\,Si(CH_3)_2OA_2$, wherein N is 1 to 100 or up to 1,000, and $A_2$ is $C_1$—$C_{30}$ alkyl, or

(C) $(CH_3)_3SiO\!-\!\!\left(Si(CH_3)\ (H)O\right)_p\!\!-\,Si(CH_3)_3$, wherein p is 1 to 100 or up to 1,000,

so that at least one Si-atom of each chain is linked to the pigment surface or the coated pigment surface through an oxygen atom,

wherein the precoated pigment comprises up to 10 wt.% of the nail enamel and

b) mixing with other nail enamel components including a base comprising nitrocellulose, solvent and a suspension agent and optionally also including a resin and a plasticizer.

7. The process according to Claim 6, wherein the polyorganosiloxane is polymethylhydrogensiloxane.

8. The process according to Claim 6 and 7, wherein the precoated pigment comprises up to about 2 wt.% of the nail enamel.

9. The process according to Claims 6 to 8, wherein the precoated pigments comprises about 1 wt.% to about 2 wt.%.

10. The process according to Claims 6 to 9, wherein the inorganic pigment is selected from iron oxide and titanium dioxide.

11. Use of an inorganic pigment precoated with polyorganosiloxane including polymeric chains having up to 100 or even to 1,000 repeating (Si—O) units, wherein the chains are cross-linked to each other, the remaining functional sites of each Si-atom in the chain are occupied by hydrogen, methyl, $C_2$—$C_{30}$ alkyl or alkenyl, and/or phenyl, resulting in units such as $-\!\left(Si(CH_3)\ (C_6H_5)O\right)\!-$, $-\!\left(Si(CH_3)\ (H)O\right)\!-$, or $-\!\left(Si(H)\ (C_6H_5)O\right)\!-$, and generally capped with $-Si(CH_3)_3$, in the preparation of a nail enamel.

12. Use of an inorganic pigment precoated with polyorganosiloxane as in Claim 11, wherein the coated pigment is formed by reacting the uncoated pigment with a silane of the formula:

$$A_1SiX_1X_2X_3,$$

wherein $A_1$ is alkyl or alkenyl having up to 30 carbon atoms, and $X_1$, $X_2$, $X_3$ are independently chloro, methoxy or ethoxy, so that at least one Si-atom of each chain is linked to the pigment surface of the coated pigment surface through an oxygen atom.

13. Use of an inorganic pigment precoated with polyorganosiloxane as in Claims 11 and 12, wherein the polyorganosiloxane is polymethylhydrogensiloxane.

14. The use of inorganic pigment precoated with polyorganosiloxane as in Claims 11 to 13, wherein the nail enamel further comprises a base comprising nitrocellulose, solvent and a suspension agent and optionally also including a resin and a plasticizer.

15. The use of inorganic pigment precoated with polyorganosiloxane as in Claims 11 to 13, wherein the precoated pigment comprises up to about 10 wt.% of the nail enamel.

16. The use of inorganic pigment precoated with polyorganosiloxane as in Claims 11 to 13, wherein the precoated pigment comprises up to about 2 wt.% of the nail enamel.

**Patentansprüche**

1. Nagellack, enthaltend

a) ein anorganisches Pigment ausgewählt aus Eisenoxiden, titanisiertem Glimmer, eisenoxidüberzogenem Glimmer. Titandioxid, Ultramarin, Chromoxid, Chromhydroxid und Manganviolett, wobei das anorganische Pigment überzogen worden ist mit einem Polyorganosiloxan mit Polymerketten mit bis zu 100 oder sogar bis 1000 wiederkehrenden (Si—O)-Einheiten, die Ketten miteinander vernetzt sind sowie die verbleibenden funktionellen Stellen an jedem Si-Atom in der Kette durch Wasserstoff, Methyl, $C_2$ bis $C_{30}$-Alkyl oder Alkenyl und/oder Phenyl unter Bildung von Einheiten wie $-\!\!\left(Si(CH_3)(C_6H_5)O\right)\!\!-$, $-\!\!\left(Si(CH_3)(H)O\right)\!\!-$ oder $-\!\!\left(Si(H)(C_6H_5O\right)\!\!-$ besetzt sind und im allgemeinen $—Si(CH_3)_3$ den Abschluß bilden, wobei ferner das Überziehen durch Reaktion des unbehandelten Pigments mit einem Siliconmaterial ausgewählt aus

(A) einem Silan der Formel $A_1SiX_1X_2X_3$, worin $A_1$ eine Alkyl oder Alkenyl mit bis zu 30 C-Atomen ist und $X_1$, $X_2$ und $X_3$ unabhängig voneinander Chlor, Methoxy oder Ethoxy bedeuten,

(b) einer Verbindung entsprechend $(CH_3)_3SiO\!-\!\!\left(Si(CH_3)_2O\right)\!\!-_nSi(CH_3)_2OA_2$, worin n 1 bis 100 oder bis zu 1000 ist und $A_2$ ein $C_1$ bis $C_{30}$-Alkyl darstellt oder

(C) einer Verbindung entsprechend $(CH_3)_3SiO\!-\!\!\left(Si(CH_3)(H)O\right)\!\!-_pSi(CH_3)_3$, worin p 1 bis 100 oder bis zu 1000 bedeutet,

erfolgt ist, so daß wenigstens ein Si-Atom jeder Kette verbunden ist mit der Oberfläche des Pigments oder des überzogenen Pigments über ein Sauerstoffatom und das überzogen Pigment in einer Menge bis zu 10 Gew.-% ein dem Nagellack vorliegt, sowie

b) eine Basis aus Nitrocellulose, Lösungsmittel und einem Schwebemittel mit wahlweisem Zusatz von einem Harz und einem Weichmacher.

2. Nagellack gemäß Patentanspruch 1, worin das Polyorganosiloxan ein Polymethylwasserstoffsiloxan ist.

3. Nagellack gemäß Patentanspruch 1 und 2, worin das überzogene Pigment in einer Menge bis zu etwa 2 Gew.-% vorliegt.

4. Nagellack gemäß den Patentansprüchen 1 bis 3, worin das überzogene Pigment in einer Menge von etwa 1 bis etwa 2 Gew.-% vorliegt.

5. Nagellack gemäß den Patentansprüchen 1 bis 4, worin das anorganische Pigment ausgewählt ist aus Eisenoxid und Titandioxid.

6. Verfahren zur Herstellung eines Nagellacks, umfassend die folgenden Stufen:

a) Überziehen eines anorganischen Pigments ausgewählt aus Eisenoxiden, titanisiertem Glimmer, eisenoxid-überzogenem Glimmer, Titandioxid, Ultramarin, Chromoxid, Chromhydroxid und Manganviolett mit einem Polyorganosiloxan mit Polymerketten von bis zu 100 oder sogar bis 1000 wiederkehrenden (SiO)-Einheiten, wobei die Ketten miteinander vernetzt sind und die verbleibenden funktionellen Stellen von jedem Si-Atom in der Kette besetzt sind durch Wasserstoff, Methyl, $C_2$ bis $C_{30}$-Alkyl oder Alkenyl und/oder Phenyl unter Bildung von Einheiten wie $-\!\!\left(Si(CH_3)(C_6H_5)O\right)\!\!-$, $-\!\!\left(Si(CH_3)(H)O\right)\!\!-$ oder $-\!\!\left(Si(H)(C_6H_5)O\right)\!\!-$ sowie $—Si(CH_3)_3$ im allgemeinen den Abschluß bilden, wobei das Überziehen durchgeführt wird durch Reaktion des unbehandelten Pigments mit einem Siliconmaterial aus

(A) einem Silan der Formel $A_1SiX_1X_2X_3$, worin $A_1$ ein Alkyl oder Alkenyl mit bis zu 30 C-Atomen ist und $X_1$, $X_2$ und $X_3$ unabhängig voneinander Chlor, Methoxy oder Ethoxy bedeuten,

(B) einer Verbindung entsprechend $(CH_3)_3SiO\!-\!\!\left(Si(CH_3)_2O\right)\!\!-_nSi(CH_3)_2OA_2$, worin n 1 bis 100 oder bis zu 1000 ist, und $A_2$ ein $C_1$ bis $C_{30}$-Alkyl bedeutet oder

(C) einer Verbindung entsprechend $(CH_3)_3SiO\!-\!\!\left(Si(CH_3)(H)O\right)\!\!-_pSi(CH_3)_3$, worin p 1 bis 100 oder bis zu 1000 ist,

so daß wenigstens ein Si-Atom einer jeden Kette verbunden ist mit der Oberfläche des Pigments oder

überzogenen Pigments über ein Sauerstoffatom und das überzogene Pigment bis zu 10 Gew.-% in dem Nagellack enthalten ist, und

b) Vermischen mit anderen Nagellack-Bestandteilen einschließlich einer Basis aus Nitrocellulose, Lösungsmittel und einem Schwebemittel mit wahlweisem Zusatz eines Harzes und eines Weichmachers.

7. Verfahren gemäß Patentansprüche 6, worin das Polysiloxan ein Polymethylwasserstoffsiloxan ist.

8. Verfahren gemäß Patentanspruch 6 und 7, worin das überzogene Pigment in einer Menge bis zu etwa 2 Gew.-% vorliegt.

9. Verfahren gemäß den Patentansprüchen 6 bis 8, worin das überzogene Pigment in einer Menge von etwa 1 bis etwa 2 Gew.-% vorliegt.

10. Verfahren gemäß den Patentansprüchen 6 bis 9, worin das anorganische Pigment ausgewählt ist aus Eisenoxid und Titandioxid.

11. Verwendung eines anorganischen Pigments, das mit einem Polyorganosiloxan mit Polymerketten von bis zu 100 oder gar bis zu 1000 wiederkehrenden Einheiten überzogen ist, wobei die Ketten miteinander vernetzt sind und die verbleibenden funktionellen Stellen von jedem Si-Atom besetzt sind mit Wasserstoff, Methyl, $C_2$ bis $C_{30}$-Alkyl oder Alkenyl und/oder Phenyl unter Bildung von Einheiten wie $-(Si(CH_3)(C_6H_5)O)-$, $-(Si(CH_3)(H)O)-$ oder $-(Si(H)(C_6H_5)O)-$ sowie $-Si(CH_3)_3$ den Abschluß bilden bei der Herstellung von einem Nagellack.

12. Verwendung eines anorganischen, mit einem Polyorganosiloxan überzogenen Pigments gemäß Patentanspruch 11, worin das überzogene Pigment hergestellt ist durch Reaktion des unbehandelten Pigments mit einem Silan gemäß der Formel $A_1SiX_1X_2X_3$, in der A ein Akyl oder Alkenyl mit bi zu 30 C-Atomen ist und $X_1$, $X_2$ und $X_3$ unabhängig voneinander Chlor, Methoxy oder Ethoxy bedeuten, so daß wenigstens ein Si-Atom einer jeden Kette mit der Oberfläche des Pigments oder überzogenen Pigments verbunden ist über ein Sauerstoffatom.

13. Verwendung eines anorganischen, mit einem Polyorganosiloxan überzogenen Pigments gemäß den Patentansprüchen 11 und 12, worin das Polyorganosiloxan ein Polymethylwasserstoffsiloxan ist.

14. Verwendung eines anorganischen, mit einem Polyorganosiloxan überzogenen Pigments gemäß den Patentansprüchen 11 bis 13, worin der Nagellack ferner enthält eine Basis aus Nitrocellulose,, Lösungsmittel und Schwebemittel mit wahlweisem Zusatz eines Harzes und eines Weichmachers.

15. Verwendung eines anorganischen, mit einem Polyorganosiloxan überzogenen Pigments gemäß den Patentansprüchen 11 bis 13, worin das überzogene Pigment in einer Menge bis zu etwa 10 Gew.-% in dem Nagellack vorliegt.

16. Verwendung eines anorganischen, mit einem Polyorganosiloxan überzogenen Pigments gemäß den Patentansprüchen 11 bis 13, worin das überzogene Pigment in einer Menge von etwa 1 bis etwa 2 Gew.-% in dem Nagellack vorliegt.

**Revendications**

1. Vernis à ongles comprenant:

(a) un pigment minéral choisi parmi les oxydes de fer, le mica titanaté, le mica enrobé d'oxyde de fer, le dioxyde de titane, l'outremer, l'oxyde de chrome, l'hydroxyde de chrome et le violet de manganèse, ledit pigment minéral étant prérenrobé d'un polyorganosiloxane incluant des chaînes polymères ayant jusqu'à 100 ou même jusquà 1000 motifs répétitifs (Si—O), les chaînes étant réticulées entre elles, les sites fonctionnels restants de chaque atome de Si dans la chaîne étant occupés par hydrogène, méthyle, alkyle ou alcényle en $C_2$—$C_{30}$ et/ou phényle, conduisant à des motifs, tels que $-(Si(CH_3(C_6H_5)O)-$, $-(Si(CH_3)(H)O)-$ ou $-(Si(H)(C_6H_5)O)-$, et généralement coiffés par $-Si(CH_3)_3$, ledit prérenrobage étant obtenu par réaction du pigment non-enrobé avec une matière de silicone choisie parmi

(A) un silane de formule $A_1SiX_1X_2X_3$, dans laquelle $A_1$ représente alkyle ou alcényle ayant jusqu'à 30 atomes de carbone et $X_1$, $X_2$ et $X_3$ représentent indépendamment chloro, mèthoxy ou éthoxy;

(B) $(CH_3)3SiO-(Si(CH_3)_2O)-_n Si(CH_3)_2OA_2$, où n vaut 1 à 100 ou jusqu'à 1000, et $A_2$ représente alkyle en $C_1$—$C_{30}$; ou

(C) $(CH_3)_3SiO(SiO-(Si(CH_3)(H)O)-_p Si(CH_3)_3$, où p vaut 1 à 100 ou jusqu'à 1000,

de telle sorte qu'au moins une atome de Si de chaque chaîne soit lié à la surface du pigment ou à la surface du pigment enrobé par l'intermédiaire d'un atome d'oxygène, le pigment préenrobé comprenant jusqu'à 10% en poids du vernis à ongles, et

(b) une base comprenant de la nitrocellulose, un solvant et un agent de mise en suspension et incluant également facultativement une résine et un plastifiant.

2. Vernis à ongles selon la revendication 1, dans lequel le polyorganosiloxane est le polyméthylhydrogénosiloxane.

3. Vernis à ongles selon l'une des revendications 1 et 2, dans lequel le pigment préenrobé représente jusqu'à environ 2% en poids du vernis à ongles.

4. Vernis à ongles selon l'une des revendications 1 à 3, dans lequel le pigment préenrobé représente d'environ 1% en poids à environ 2% en poids.

5. Vernis à ongles selon l'une des revendications 1 à 4, dans lequel le pigment minéral est choisi parmi l'oxyde de fer et le dioxyde de titane.

6. Procédé de préparation d'un vernis à ongles comprenant les étapes consistant:

a) à réaliser le préenrobage d'un pigement minéral choisi parmi les oxydes de fer, le mica titanaté, le mica enrobé par de l'oxyde de fer, le dioxyde de titane, l'outremer, l'oxyde de chrome, l'hydroxyde de chrome et le violet de manganèse, par un polyorganosiloxane incluant des chaînes polymères ayant jusqu'à 100 ou même jusqu'à 1000 motifs répétitifs (Si—O), les chaînes étant réticulées entre elles, les sites fonctionnels restants de chaque atome de Si dans la chaîne étant occupés par hydrogène, méthyle, alkyle ou alcényle en $C_2$—$C_{30}$ et/ou phényle, conduisant à des motifs, tels que $-(-Si(CH_3(C_6H_5)O-)-$, $-(-Si(CH_3)(H)O-)-$, ou $-(-Si(H)(C_6H_5O-)-$, et généralement coiffés par $—Si(CH_3)_3$, ledit préenrobage étant obtenu par réaction du pigment non-enrobé avec une matière de type silicone choisie parmi

(A) un silane de formule $A_1SiX_1X_2X_3$, dans laquelle $A_1$ représente alkyle ou alcényle ayant jusqu'à 30 atomes de carbone et $X_1$, $X_2$ et $X_3$ représentent indépendamment chloro, méthoxy ou éthoxy;

(B) $(CH_3)_3SiO-(-Si(CH_3)_2O-)_n$ $Si(CH_3)_2OA_2$, où n vaut 1 à 100 ou jusqu'à 1000, et $A_2$ représente alkyle en $C_1$—$C_{30}$; ou

(C) $(CH_3)_3SiO-(-Si(CH_3)(H)O-)_p$ $Si(CH_3)_3$, où p vaut 1 à 100 ou jusqu'à 1000,

de telle sorte qu'au moins un atome de Si de chaque chaîne soit lié à la surface du pigment ou à la surface du pigment revêtu par l'intermédiaire d'un atome d'oxygène,

le pigment préenrobé représentant jusqu'à 10% en poids du vernis à ongles, et

b) à mélanger avec d'autres composants de vernis à ongles incluant une base comprenant de la nitrocellulose, un solvant et un agent de mise en suspension et incluant également facultativement une résine et un plastifiant.

7. Procédé selon la revendication 6, dans lequel le polyorganosiloxane est le polyméthylhydrogénosiloxane.

8. Procédé selon l'une des revendications 6 et 7, dans lequel le pigment préenrobé représente jusqu'à environ 2% en poids du vernis à ongles.

9. Procédé selon l'une des revendications 6 à 8, dans lequel le pigment préenrobé représente d'environ 1% en poids à environ 2% en poids.

10. Procédé selon l'une des revendications 6 à 9, dans lequel le pigment minéral est choisi parmi l'oxyde de fer et le dioxyde de titane.

11. Utilisation d'un pigment minéral préenrobé d'un polyorganosiloxane incluant les chaînes polymères ayant jusqu'à 100 ou même jusqu'à 1000 motifs répétitifs (Si—O), les chaînes étant réticulées entre elles, les sites fonctionnels restants de chaque atome de Si dans la chaîne étant occupés par hydrogène, méthyle, alkyle ou alcényle en $C_2$—$C_{30}$ et/ou phényle, conduisant à des motifs tels que $-(-Si(CH_3)$ $(C_6H_5)O-)-$, $-(-Si(CH_3)(H)O-)-$, ou $-(-Si(H)(C_6H_5)O-)-$, et généralement coiffés par $—Si(CH_3)_3$, dans la préparation d'un vernis à ongles.

12. Utilisation d'un pigment minéral préenrobé de polyorganosiloxane selon la revendication 11, dans laquelle le pigment enrobé est formée par réaction du pigment non-enrobé avec un silane de formule:

$$A_1SiX_2X_2X_3,$$

dans laquelle:

$A_1$ représente alkyle ou alcényle ayant jusqu'à 30 atomes de carbone; et

$X_1$, $X_2$, $X_3$ représentant indépendamment chloro, méthoxy ou éthoxy.

de telle sorte qu'au moins un atome de Si de chaque chaîne soit lié à la surface du pigment ou à la surface du pigment enrobé par l'intermédiaire d'un atome d'oxygène.

13. Utilisation d'un pigment minéral préenrobé de polyorganosiloxane selon l'une des revendications 11 et 12, dans laquelle le polyorganosiloxane est le polyméthylehydrogénosiloxane.

14. Utilisation de pigment minéral préenrobé de polyorganosiloxane selon l'une des revendications 11 à 13, dans laquelle le vernis à ongles comprend en outre une base comprenant de la nitrocellulose, un solvant et un agent de mise en suspension et incluant aussi, de façon facultative, une résine et un plastifiant.

15. Utilisation de pigment minéral préenrobé de polyorganosiloxane selon l'une des revendications 11 à 13, dans laquelle le pigment préenrobé représente jusqu'à environ 10% en poids du vernis à ongles.

16. Utilisation du pigment minéral préenrobé de polyorganosiloxane selon l'une des revendications 11 à 13, dans laquel le pigment préenrobé représente jusqu'à environ 2% en poids du vernis à ongles.